# EUROPEAN PATENT APPLICATION

(11) **EP 3 903 693 A1**
(43) Date of publication of application: **03.11.2021**
(21) Application number: 19903405.9
(22) Date of filing: 20.12.2019
(51) Int. Cl.: A61B 17/068

(54) **SAFETY DEVICE FOR SURGICAL INSTRUMENT**

(30) Priority: 25.12.2018 CN 201811589772
(71) Applicant: Ezisurg Medical Co., Ltd., Shanghai 201203 (CN); Ezisurg (Suzhou) Medical Co., Ltd., Suzhou, Jiangsu 215163 (CN)
(72) Inventor: YANG, Guang, Shanghai 201318 (CN); NIE, Honglin, Shanghai 201318 (CN)
(74) Representative: Mansfield, Peter Turquand
(86) International application number: PCT/CN2019/126973
(87) International publication number: WO 2020/135253

(57) **Abstract**

A surgical instrument (1) with a safety mechanism includes an end effector, an intermediate connector, and a controller (100), wherein the controller (100) includes housings (101, 102), a drive mechanism, the safety mechanism, and a reset mechanism, the drive mechanism, the safety mechanism and the reset mechanism being arranged or partially arranged in the housings (101, 102); the safety mechanism includes a safety trigger (104), a dry firing function block (109), and a biasing member (110). The surgical instrument can solve the problem that the instrument cannot be fired and reset when an automatic reset mechanism thereof fails, thereby protecting patient safety, and having a simple structure and a low cost.

## Description

### TECHNICAL FIELD

The present invention relates to a surgical operation instrument, and more particularly to a safe mechanism for a surgical stapler.

### BACKGROUND

The operating principle of a surgical stapler is to clamp tissues by closing two corresponding jaws (which are generally called an anvil assembly and a staple cartridge assembly), and then pushing out metal suturing staples from the staple cartridge of the stapler to suture the tissues together. In some staplers, a cutting knife is also mounted, and is used to incise the sutured tissues. A stapler having the above functions includes an end effector, an intermediate connector, and a controller, wherein the end effector consists of an anvil assembly, a staple cartridge assembly, and a drive assembly. The anvil assembly includes a staple forming surface which is provided with multiple rows of staple pockets for forming metal suturing staples. The staple cartridge assembly generally includes a staple cartridge, suturing staples, staple drivers, a staple pushing slider, and a staple cartridge base; an upper surface of the staple cartridge is a tissue contact surface; and the staple cartridge is mounted in the staple cartridge base. A proximal end of the anvil assembly is movably connected to a proximal end of the staple cartridge assembly, and can switch between an open state and a closed state. Generally, the anvil assembly and the staple cartridge base further respectively include a longitudinal channel for the drive assembly to pass. When the drive assembly moves to a distal end of the end effector via the longitudinal channel, the anvil assembly and the staple cartridge assembly are driven to switch from the open state to the closed state, and the staple pushing slider and the staple drivers are driven to push out the suturing staples which are then formed in the staple pockets on the staple forming surface of the anvil assembly.

Generally, the drive assembly further includes a cutting knife, wherein the cutting knife is used to cut the tissue between multiple rows of staple lines after the tissue is sutured with the suturing staples. The controller is used to manually control and operate the instrument, and generally includes a fixed handle, a firing trigger which is relatively movably connected to the fixed handle, a drive mechanism for transferring an action of the firing trigger to the end effector, and a manual reset mechanism. The drive mechanism is connected to the drive assembly, and is used to convert the action of the firing trigger into the actions of the end effector such as closing, firing, opening and the like. When the staple cartridge assembly and the anvil assembly of the end effector are in the closed state, the manual reset mechanism can be operated to drive the staple cartridge assembly and the anvil assembly to restore the open state. The intermediate connector is movably connected to a distal end of the controller, and is connected to a proximal end of the end effector; the intermediate connector forms a connection channel for transferring the action of the firing trigger to the end effector.

In many designs, the drive mechanism includes a toothed rack provided with multiple sets of teeth, wherein the first set of teeth is used to control the closing of an end effector jaw, and the second set of teeth is used to control the formation of the stapler suturing staples and the incision of the tissue. Furthermore, the stapler generally further includes a safety device; after the firing trigger is operated to close the end effector jaw, the safety device needs to be operated such that the instrument can be fired normally, otherwise the instrument cannot be fired. The safety device can prevent the stapler from being fired by mistake due to a mis-operation in a situation that a doctor does not intend.

CN103767751B discloses a surgical instrument operable with one hand. If the safety mechanism is not initiated, the staple cartridge assembly and the anvil assembly restore the open state by means of an automatic reset mechanism; the automatic reset mechanism includes an elastic energy storage assembly which is generally various springs.

The automatic reset mechanism further includes teeth which can be separably engaged with the drive assembly.

In certain specific situations (for example, if the tissue clamped by the jaw is too thick), friction forces between the drive assembly and the staple cartridge assembly as well as the anvil assembly are greater than the energy stored by the automatic reset mechanism, such that the jaw cannot open; if the firing trigger mechanism is operated again, a driving pawl of the firing trigger mechanism would be embedded in the second set of teeth of the drive assembly, in which case the firing trigger mechanism is in a half-firing state, and the engaged teeth of automatic reset mechanism cannot disengage from the drive assembly by operating the firing safety mechanism; therefore, the firing trigger mechanism cannot continuously fire, and the automatic reset mechanism cannot reset, thereby causing inconvenience to an operation. In more serious cases, an extra intervention means may be required to solve the problem.

### SUMMARY

In order to solve the technical problem in the prior art that the automatic reset mechanism cannot reset, the present invention provides a safety mechanism for a surgical stapler, the surgical stapler including an end effector, an intermediate connector, and a controller which are sequentially connected, wherein the controller includes housings, and a drive mechanism, a safety mechanism, and a reset mechanism which are arranged or partially arranged in the housings; and the safety mechanism includes a safety trigger, a dry firing function block, and a biasing member. In some embodiments, the drive mechanism includes a firing trigger, and a driving pawl and a toothed rack which are arranged in the firing trigger; the biasing member keeps the dry firing function block away from the toothed rack; and when the safety mechanism is not initiated, the dry firing function block limits the engagement between the driving pawl and the toothed rack. In some embodiments, the toothed rack includes at least two sets of teeth; the biasing member keeps the dry firing function block away from the second set of teeth of the toothed rack; and when the safety mechanism is not armed, the dry firing function block limits the engagement between the driving pawl and the second set of teeth.

In some embodiments, the surgical instrument includes an automatic reset mechanism; the automatic reset mechanism includes a toothed rack limit slider; when the firing trigger is operated, the toothed rack drives the toothed rack limit slider to move forward, until the toothed rack limit slider pushes the dry firing function block to overcome the force of the biasing member and move; and the dry firing function block limits the engagement between the driving pawl and the second set of teeth.

In some embodiments, when the safety trigger is operated, the dry firing function block can move away from the second set of teeth, so as to release the limited engagement between the driving pawl and the second set of teeth.

In some embodiments, when the safety trigger is operated, the toothed rack limit slider is detached from the dry firing function block, and the dry firing function block keeps away from the second set of teeth under the action of the biasing member, so as to release the limited engagement between the driving pawl and the second set of teeth.

In some embodiments, the dry firing function block includes a shielding structure; when the dry firing function block moves adjacent to the second set of teeth of the toothed rack, the shielding structure shields the second set of teeth of the toothed rack with the movement of the dry firing function block; and when the dry firing function block is away from the second set of teeth, the shielding of the second set of teeth by the shielding structure is released.

In some embodiments, the shielding structure has a sheet-like shape, or a block-like shape, or a strip shape.

In some embodiments, the dry firing function block rotates about an axis.

In some embodiments, the dry firing function block makes a rectilinear or curvilinear motion along a specific guide rail.

In some embodiments, the biasing member is an elastic member.

In some embodiments, the biasing member is one of an elastic sheet, a tension spring, a pressure spring, and a torsion spring.

The safety mechanism for the surgical stapler of the present invention can solve the problem that the instrument cannot be fired and reset when the automatic reset mechanism thereof fails. When the safety mechanism of the instrument is not initiated, the driving pawl cannot be embedded in the second set of teeth of the toothed rack, in which case the instrument cannot be fired, thereby protecting patient safety. Furthermore, the surgical stapler of the present invention has a simple structure and a low cost.

### BRIEF DESCRIPTION OF THE DRAWINGS

The drawings are provided for further understanding the present invention, constitute a part of the specification, and are used, together with specific embodiments of the present invention, to explain the present invention, but are not intended to limit the present invention.
Fig. 1 is a view of internal components in an original state in a surgical stapler according to one embodiment of the present invention;
Fig. 2 is a vertical view of a controller of the surgical stapler in Fig. 1;
Fig. 3 is sectional view of a part of the controller in Fig. 2, and shows the original state of the internal components;
Fig. 4 is a view of the internal components after a firing trigger is operated in the surgical stapler according to one embodiment of the present invention;
Fig. 5 is a view of the internal components before a safety mechanism is initiated in the surgical stapler according to one embodiment of the present invention;
Fig. 6 is a view of the internal components after the safety mechanism is initiated in the surgical stapler according to one embodiment of the present invention;
Fig. 7 is a dry firing function block and an elastic sheet according to one embodiment of the present invention;
Fig. 8 is a toothed rack limit slider according to one embodiment of the present invention;
Fig. 9 is a toothed rack according to one embodiment of the present invention; and
Fig. 10 is assembly relationships of a left housing, the dry firing function block, and the elastic sheet according to one embodiment of the present invention.

### DETAILED DESCRIPTION OF THE EMBODIMENTS

The present invention will be further described hereafter with reference to specific embodiments, wherein the drawings are used for illustrative purpose only, denote schematic views only rather than physical views, and thus should not be considered as a limitation to the present patent. In order to describe the specific embodiments of the present invention still better, certain components in the drawings may be omitted, enlarged or minified, and do not denote the sizes of a practical product. For a person skilled in the art, omitting certain commonly known structures in the drawings and the descriptions thereof is understandable. On the basis of the specific embodiments of the present invention, all the other specific embodiments obtained by a person skilled in the art without involving an inventive effort are included in the protection scope of the present invention.

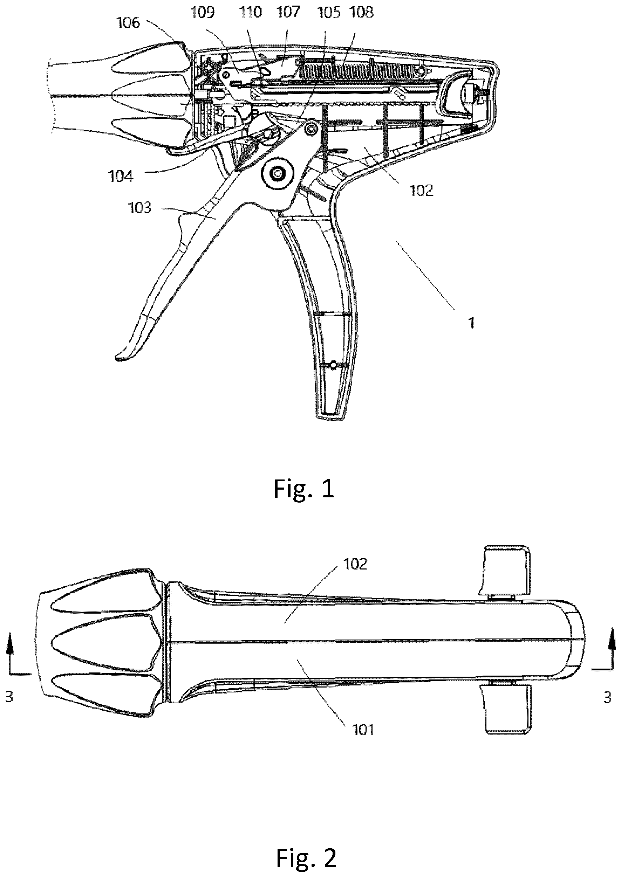

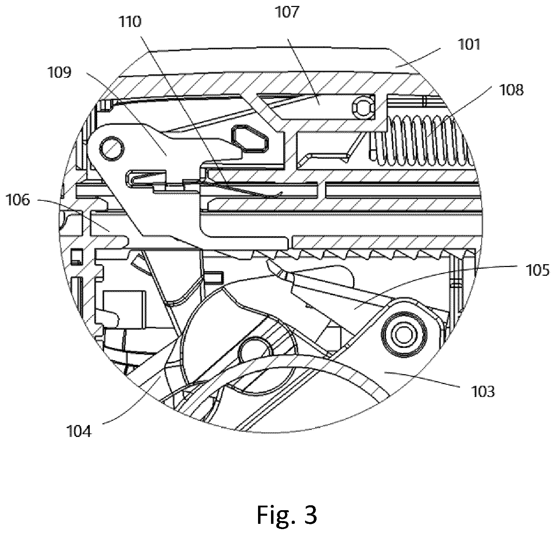

Figures 1-10 show schematic views of the stapler or components thereof according to one embodiment of the present invention.

Specifically, in the present embodiment, with reference to figures 1 and 4-6, the surgical stapler 1 of the present invention includes a controller 100, an intermediate connector, and an end effector (wherein the intermediate connector and the end effector are conventional structural form in the existing surgical stapler, and are therefore not specifically shown). The controller 100, the intermediate connector, and the end effector are sequentially connected end-to-end. The end effector includes an anvil assembly, a staple cartridge assembly, and a drive assembly, wherein the anvil assembly and the staple cartridge assembly form a jaw which can switch between an open state and a closed state; the drive assembly controls the opening and closing of the jaw, and pushes out anastomosis staples from the staple cartridge to form at a tissue contact surface of the anvil.

With reference to figures 1-6, the controller 100 includes a left housing 101, a right housing 102, a firing trigger 103, a safety trigger 104, a driving pawl 105, a toothed rack 106, a toothed rack limit slider 107, a tension spring 108, a dry firing function block 109, and an elastic sheet 110, wherein the firing trigger 103 is partially arranged in the left housing 101 and the right housing 102, and can rotate about a fixed rotary shaft; the driving pawl 105 is mounted on the firing trigger 103, and can rotate about a movable rotary shaft; and the driving pawl 105 can be separably engaged with a first set of teeth 1061 or a second set of teeth 1062 of the toothed rack 106.

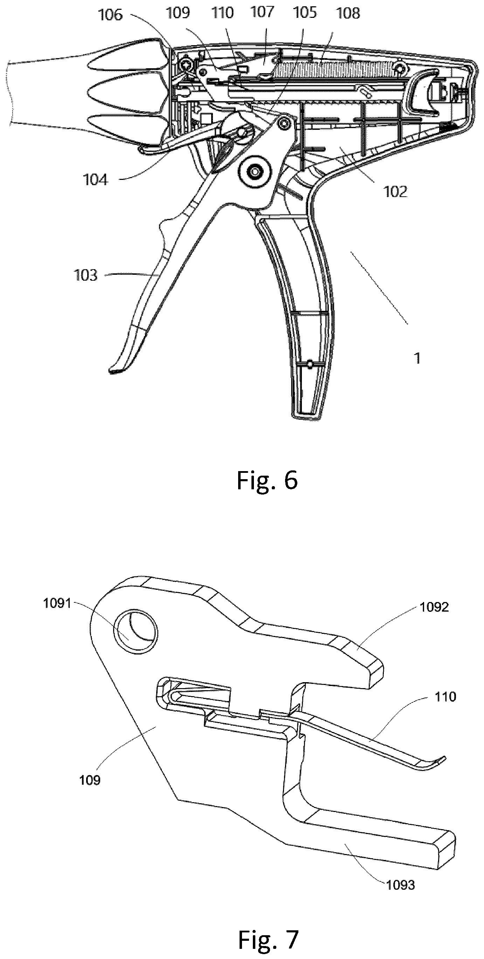

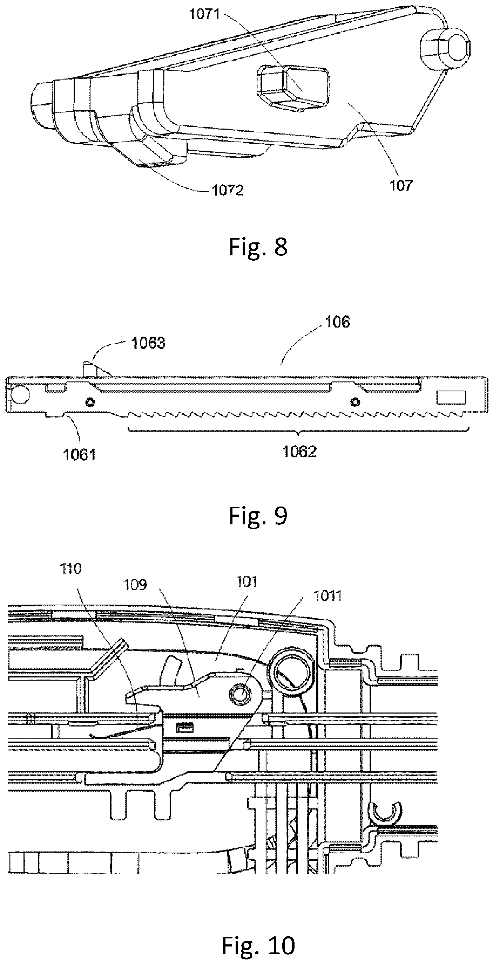

With reference to figures 7 and 9-10, a circular hole 1091 is disposed on the dry firing function block 109 (figure 7); the circular hole is matched with a cylinder 1011 located at the left housing 101, and can rotate a certain angle about the cylinder 1011 (figure 10). One end of the elastic sheet 110 acts on the dry firing function block 109, and the other end acts on the left housing 101; the elastic sheet 110 can enable a shielding structure 1093 located at the dry firing function block 109 to lean to a direction away from the second set of teeth 1062 of the toothed rack 106 (figure 9).

In other embodiments, the dry firing function block 109 can slide along a guide in a direction away from the second set of teeth 1062 of the toothed rack 106; the elastic sheet 110 can also be a spring, a torsion spring, or other elastic components, and acts on the left housing 101, or the right housing 102, or other components.

With reference to figure 8, the toothed rack limit slider 107 is provided with a protruding platform 1071 (figure 8), and the protruding platform 1071 is matched with an inclined surface 1092 (figures 7 and 10) of the dry firing function block 109.

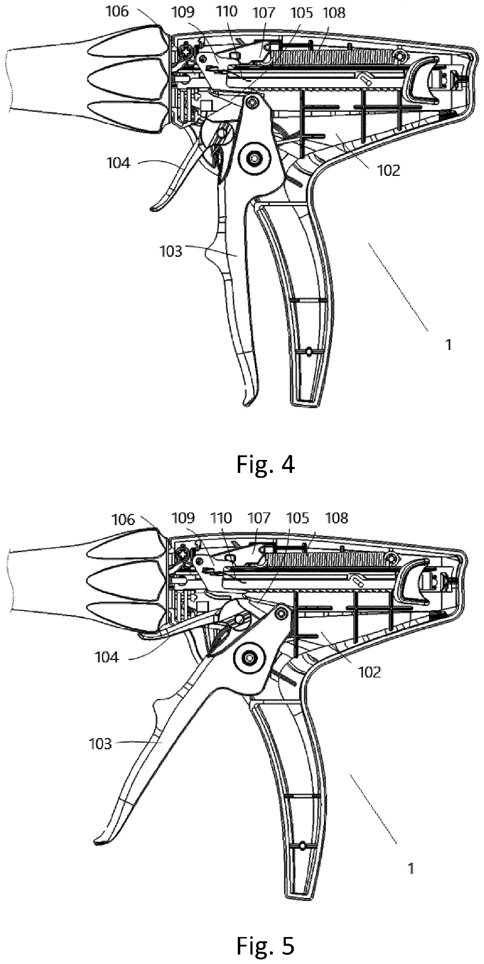

The specific operations of the present embodiment are as follows. As shown in figure 4, the firing trigger 103 is rotated by a certain angle; the rotary driving pawl 105 on the firing trigger 103 is engaged with the first set of teeth 1061 of the toothed rack 106, so as to push the toothed rack 106 to move forward a certain distance. A protruding platform 1063 detachably connected to the toothed rack limit slider 107 is disposed on the toothed rack 106; the protruding platform 1063 acts on a protruding platform 1072 arranged on the toothed rack limit slider 107, and drives the toothed rack limit slider 107 to move forward. The toothed rack limit slider 107 contacts the inclined surface 1092 of the dry firing function block 109 via the protruding platform 1071 on a lateral surface, such that the dry firing function block 109 overcomes an elastic force of the elastic sheet 110 to rotate; the dry firing function block 109 rotates a certain angle; and the shielding structure 1093 shields the second set of teeth 1062 of the toothed rack 106.

As shown in figure 5, the firing trigger 103 is released, and the driving pawl 105 is disengaged with the first set of teeth 1061 of the toothed rack 106. If friction forces between the drive assembly and the staple cartridge assembly as well as the anvil assembly are greater than the energy stored by the tension spring 108, the tension spring 108 cannot reset the toothed rack 106 via the toothed rack limit slider 107, and the jaw cannot open, then if the firing trigger 103 is operated again, the driving pawl 105 would not be engaged with the second set of teeth 1062 of the toothed rack 106 and cannot push the toothed rack 106 to move forward because the shielding structure 1093 of the dry firing function block 109 shields the second set of teeth 1062 of the toothed rack 106.

As shown in figure 6, the safety trigger 104 is operated to disengage the toothed rack limit slider 107 from the toothed rack 106 to reset the toothed rack limit slider 107. The protruding platform 1071 of the toothed rack limit slider 107 does not contact the inclined surface 1092 of the dry firing function block 109; under the action of the elastic sheet 110, the shielding structure 1093 of the dry firing function block 109 gets away from the second set of teeth 1062 of the toothed rack 106. The firing trigger 103 is operated again; and the driving pawl 105 can be engaged with the second set of teeth 1062 of the toothed rack 106, so as to push the toothed rack 106 to move forward to suture a cut.

Although the specific embodiments of the present invention have been shown and described, it can be understood that a person skilled in the art can make various changes, modifications, substitutions and variations to the specific embodiments without departing from the principle and spirit of the present invention; and the scope of the present invention is defined by the attached claims and equivalents thereof.

## Claims

1. A surgical instrument, **characterized in that** the surgical instrument comprises an end effector, an intermediate connector and a controller which are sequentially connected, wherein the controller comprises housings, and a drive mechanism and a safety mechanism which are arranged or partially arranged in the housings; and
the safety mechanism comprises a safety trigger, a dry firing function block, and a biasing member.

2. The surgical instrument according to claim 1, **characterized in that** the drive mechanism comprises a firing trigger, and a driving pawl and a toothed rack which are arranged in the firing trigger; the biasing member keeps the dry firing function block away from the toothed rack; and when the safety mechanism is not initiated, the dry firing function block limits the engagement between the driving pawl and the toothed rack.

3. The surgical instrument according to claim 2, **characterized in that** the toothed rack comprises a first set of teeth and a second set of teeth; the biasing member keeps the dry firing function block away from the second set of teeth of the toothed rack; and when the safety mechanism is not initiated, the dry firing function block limits the engagement between the driving pawl and the second set of teeth.

4. The surgical instrument according to claim 3, **characterized in that** the surgical instrument comprises an automatic reset mechanism; the automatic reset mechanism comprises a toothed rack limit slider; when the firing trigger is operated, the toothed rack drives the toothed rack limit slider to move forward, until the toothed rack limit slider pushes the dry firing function block to overcome a force of the biasing member and move; and the dry firing function block limits the engagement between the driving pawl and the second set of teeth.

5. The surgical instrument according to claim 3, **characterized in that** the safety trigger is operated, the dry firing function block can move away from the second set of teeth, so as to release the limited engagement between the driving pawl and the second set of teeth.

6. The surgical instrument according to claim 4, **characterized in that** when the safety trigger is operated, the toothed rack limit slider is detached from the dry firing function block, and the dry firing function block keeps away from the second set of teeth under the action of the biasing member, so as to release the limited engagement between the driving pawl and the second set of teeth.

7. The surgical instrument according to claim 3, **characterized in that** the dry firing function block comprises a shielding structure; when the dry firing function block moves adjacent to the second set of teeth of the toothed rack, the shielding structure shields the second set of teeth of the toothed rack with the movement of the dry firing function block; and when the dry firing function block is away from the second set of teeth, the shielding of the second set of teeth by the shielding structure is released.

8. The surgical instrument according to claim 7, **characterized in that** the shielding structure has a sheet-like shape, or a block-like shape, or a strip shape.

9. The surgical instrument according to claim 1, **characterized in that** the dry firing function block rotates about an axis.

10. The surgical instrument according to claim 1, **characterized in that** the dry firing function block makes a rectilinear or curvilinear motion along a specific guide rail.

11. The surgical instrument according to claim 1, **characterized in that** the biasing member is an elastic member.

12. The surgical instrument according to claim 11, **characterized in that** the biasing member is one of an elastic sheet, a tension spring, a pressure spring, and a torsion spring.
